# EUROPEAN PATENT SPECIFICATION

(11) **EP 4 291 093 B1**
(45) Date of publication and mention of the grant of the patent: **20.11.2024**
(21) Application number: 22709675.7
(22) Date of filing: 25.02.2022
(51) Int. Cl.: A61B 5/20, G01F 23/26, G01F 23/263, G01F 23/80

(54) **DEVICE FOR TRACKING URINE OUTPUT**
VORRICHTUNG ZUR VERFOLGUNG DER URINMENGE
DISPOSITIF DE SUIVI DE DIURÈSE

(30) Priority: 26.02.2021 EP 21159677
(43) Date of publication of application: 20.12.2023
(73) Proprietor: Minze NV, 2000 Antwerpen (BE)
(72) Inventor: SAGEDER, Josef, 2018 Antwerpen (BE); BLADT, Lola, 2000 Antwerpen (BE); VERMEULEN, Jiri, 2060 Antwerpen (BE)
(74) Representative: De Clercq & Partners
(86) International application number: PCT/EP2022/054863
(87) International publication number: WO 2022/180239

(56) References cited:
- WO-A1-2014/141234
- US-A- 6 125 696
- US-A1- 2017 105 670

## Description

### FIELD OF THE INVENTION

The present invention is in the field of devices for evaluating urological functions. In particular, the present invention relates to a device for tracking a daily urine output.

### BACKGROUND

The tracking of daily urine output can be important for a variety of reasons. Irregularities in urine volume can signal that a person is suffering from abnormal health conditions. The art describes that urine volume can be tracked by measuring the fluid volume in collection containers e.g. beakers after filling, and then manually entering the measured volume into a tracking table. The collection containers can be provided with visual marking to indicate the corresponding urine volume. For obvious reasons, this tracking method is tedious and time-consuming. Also, when entering measurement data post-collection human errors can occur on the exact time of voiding and precise urine volume.

Tracking urine output in a private setting such as at home opposed to in a clinical setting can provide for more reliable tracking data since it accounts for variations in urine output throughout the day. In addition to the obvious disadvantage of inconvenience, one complication that often arises with in-clinic testing is that the measurements are not representative of regular urine output which can then skew or negate the expected values and delay the detection of problems. As such, patients are typically asked to adamantly track urine output at home after every voiding. However, for the reasons discussed above there is a low patient compliance. Moreover, there is no way to verify if the patient has entered the correct values into the tracking table.

Development of reliable urine tracking devices is difficult because urine is unique; e.g., urine may be voided at different body and room temperatures, at different flow rates, having different salt concentration, and so on. For example, EP 2 456 353 describes a disposable cup for home use that is provided with various sensors to measure different urine properties and automatically timestamp the measured data. Nonetheless, the arrangement of the disposable cup makes it unsuitable for repeated use as it suffers from sensor degradation. As such, the cup needs to be replaced with every use which is not economic for prolonged home usage, especially when dealing with health conditions that require frequent voiding. Moreover, the measurement requires the user to connect the disposable cup to an external data processing module which adds to the complexity of the device with a potential risk of user error.

Furthermore US 2017/105670 A1 describes a urine measurement device comprising a capacitive sensing device and an electrical shield positioned above the capacitive sensing device to partition a sensing portion of the measurement device from a non-sensing portion of the measurement device. This electrical shield may also provide for a grounding plane for the capacitive sensing device. US 6 125 696 A describes an apparatus for detecting a level of a liquid-based fluid such as urine, the apparatus comprising: a capacitive element array including a plurality of independent input plates spaced longitudinally along an axis of measurement. WO 2014/141234 A1 describes a system for measuring a volume and flow rate of urine, which comprises a bottom capacitor and a plurality of ring capacitors.

There is a need in the art to provide a reliable and economic solution for tracking of urine output.

### SUMMARY OF THE INVENTION

The invention is defined by the appended claims.

To provide a solution to the above-identified problems in the art, the present disclosure describes a device for tracking the daily urine output of a subject. The present device comprises a capacitive sensor circuit that is configured to measure the height of the fluid filled into the receptacle and therefore allows for highly accurate urine volume measurements to be obtained irrespective of the urine's variable dielectric properties, which may vary due to differences in e.g. temperature, salt concentration, etc. Also, the device may be configured to automatically timestamp and record the urine volume to improve the tracking accuracy.

An initial overview of various aspect of the invention is provided below and specific embodiments are then described in further detail. This initial overview is intended to aid readers in understanding the technological concepts more quickly, but is not intended to identify key or essential features thereof, nor is it intended to limit the scope of the present subject-matter.

An aspect of the present disclosure relates to a device for tracking a subject's daily urine output comprising:
- a receptacle having a bottom surface and sidewalls defining an open-ended inner volume for the filling of a fluid, said open end being a top of the receptacle;
- a capacitive sensor circuit having a capacitive sensing area configured for measuring the height of the fluid filled into the receptacle, comprising two or more capacitive surface sensor electrodes that have substantially the same surface area; wherein the capacitive surface sensor electrodes are disposed along at least one sidewall of the receptacle at substantially different distances from the bottom surface such that the capacitive sensing area extends substantially upwards from the bottom surface; and wherein the capacitive sensor circuit is grounded to the fluid filled into the receptacle;
- an electrical shielding configured to enclose the capacitive surface sensor electrodes; and,
- a processing unit connected to the capacitive sensor circuit and configured to
   - receive fluid height data from the capacitive sensor circuit;
   - determine, from said fluid height data, a corresponding urine volume;
   - store said urine volume on a memory unit.

The invention relates to a device for tracking a subject's daily urine output comprising:
- a receptacle having a bottom surface and sidewalls defining an open-ended inner volume for the filling of a fluid, said open end being a top of the receptacle;
- a capacitive sensor circuit configured for measuring the height of the fluid filled into the receptacle within
- a capacitive sensing area, said sensor circuit comprising a sensor plane, at least two capacitive surface sensor electrodes coupled to the sensor plane, and a ground plane; wherein the capacitive surface sensor electrodes have substantially the same surface area and are arranged along at least one sidewall of the receptacle at substantially different distances from the bottom surface such that the capacitive sensing area extends substantially upwards from the bottom surface; and
- an electrical shielding sandwiched between the sensor plane and the ground plane; wherein the electrical shielding fully encloses the sensor plane apart from the capacitive surface sensor electrodes such that it shields the capacitive sensing area from interference and/or minimizes parasitic capacitance between the sensor plane (22) and the ground plane (23); and,
- a processing unit connected to the capacitive sensor circuit and configured to
   - receive fluid height data from the capacitive sensor circuit;
   - determine, from said fluid height data, a corresponding urine volume;
   - store said urine volume on a memory unit.

In some embodiments the capacitive surface sensor electrodes are arranged such that at least a portion of the surface area of two adjacent sensor electrodes overlaps on the same distance from the bottom of the receptacle.

In some embodiments the receptacle's inner volume is tapered towards the bottom surface.

In some embodiments the capacitive sensor circuit has a sensor plane and an opposing ground plane; wherein the capacitive surface sensor electrodes are coupled to the sensor plane; and wherein the electrical shield forms a layer between the sensor plane and the ground plane.

In some embodiments the device comprises a sensor plane and an opposite ground plane.

In some embodiments the capacitive electrodes are coupled to the sensor plane.

In some embodiments the electrical shielding is actively driven by a shield signal.

In some embodiments the electrical shielding is actively driven by a shield signal which corresponds to the input signal of the capacitive surface sensor electrodes and/or a copy thereof.

In some embodiments the capacitive surface sensor electrodes are coupled to the sensor plane with electrical connectors of substantially the same length.

In some embodiments the capacitive sensor circuit comprises a conductive element connecting the receptacle's inner volume to the ground plane; preferably wherein the conductive element comprises a conductive fastening element, such as a bolt, provided with a conductive cap.

In some embodiments the ground plane is cross-hatched or hatched.

In some embodiments the processing unit is configured to assign a timestamp to the determined urine volume; and store said timestamped urine volume on a memory unit.

In some embodiments the processing unit is configured to determine the urine volume by looking up the corresponding value in a calibration curve and/or look-up table that describes the relationship between the fluid height and the receptacle's inner volume.

In some embodiments the urine tracking device comprises a reference sensor configured to detect the presence of fluid in the receptacle's inner volume; and wherein the processing unit is configured to distinguish between a completely empty receptacle and a completely full receptacle based on measurements made by said reference sensor.

In some embodiments the urine tracking device comprises a reference capacitive sensor electrode connected to the capacitive sensor circuit that is configured to determine a baseline value independent of the presence of a fluid in the receptacle; wherein the processing unit is configured to receive the baseline value from said reference capacitive electrode and determine the presence and/or absence of fluid in the receptacle by comparing said baseline value with a capacitance value measured by one or more capacitive surface sensor electrodes; preferably wherein the reference capacitive sensor electrode is disposed between the capacitive surface sensor electrodes and the electrical shielding.

In some embodiments the urine tracking device comprises a fluid stability sensor configured for determining the stability of the fluid within the receptacle and wherein the processing unit is configured to correct the fluid height data by delaying and/or repeating the fluid height measurement by the capacitive sensor circuit

In some embodiments the processing unit is configured to provide user-feedback when fluid instability is detected to stabilise the receptacle for measurement; preferably with an audio and/or visual cue.

In some embodiments the urine tracking device comprises a tilt sensor configured for measuring the tilt of the receptacle and wherein the processing unit is configured to correct the fluid height data by determining the tilt angle and/or direction and converting the fluid height data into a corrected fluid height data.

In some embodiments the urine tracking device comprises a communication module that is configured to connect to a computing device, such as a smartphone or personal computer, and sending the determined urine volume, preferably the timestamped urine volume, to said computing device; and wherein the urine tracking device comprises a subject identification module that is configured to identify the subject when connecting the urine tracking device to the computing device.

In some embodiments the receptacle has a recessed section along one of its sidewalls with a depth that is sufficient to fully fit the capacitive sensor circuit; and wherein the device comprises a cover to secure the capacitive sensor circuit within said recess to the receptacle.

In some embodiments the device comprises one or more sealing elements disposed between the receptacle and the cover so that fluid cannot reach the capacitive sensor circuit.

An aspect of the present invention relates to a use of the device according to the present invention for tracking a subject's daily urine output.

### BRIEF DESCRIPTION OF THE FIGURES

The following description of the figures of specific embodiments of the invention are merely exemplary in nature and is not intended to limit the present teachings, their application or uses.

Throughout the drawings, the corresponding reference numerals indicate the following parts and features: receptacle 1; bottom 11; open end 12; recessed section 13; funnel 14; rim 15; capacitive sensor circuit 2; capacitive surface sensor electrode 21; sensor plane 22; ground plane 23; conductive element 24; conductive cap 25; electrical connector 26; cover 3; first sealing element 31; second sealing element 32; third sealing element 33; electrical shielding 4; electronic module 5; user interface module 6; urine tracking device 10.
**Figure 1** is an exploded view of an embodiment of the urine tracking device 10.
**Figure 2** is a front view of an embodiment of the urine tracking device 10 showing a first exemplary embodiment of the capacitive surface sensor electrodes 21.
**Figure** 3 is a side view of an embodiment of the urine tracking device 10.
**Figure 4** is a top view of an embodiment of the urine tracking device 10.
**Figure 5** is a bottom view of an embodiment of the urine tracking device 10.
**Figure 6** is a front view of the urine tracking device 10 showing a second exemplary embodiment of the capacitive surface sensor electrodes 21.
**Figure 7** is a front view of the urine tracking device 10 showing a third exemplary embodiment of the capacitive surface sensor electrodes 21.
**Figure 8** is a perspective view of an embodiment of the receptacle 1.
**Figure 9** is a perspective view of an embodiment of the receptacle 1.
**Figure 10** is a perspective view of an embodiment of the receptacle 1.
**Figure 11** is a perspective view of an embodiment of the receptacle 1.
**Figure 12A** is a front view of an empty urine tracking device 10.
**Figure 12B** is a cross-section of the urine tracking device 10 of **Figure 12A****.**
**Figure 13A** is a front view of a urine tracking device 10 which filled with fluid.
**Figure 13B** is a cross-section of the urine tracking device 10 of **Figure 13A****.**
**Figure 14** is a graph illustrating the capacitance resolution of a urine tracking device 10 defined as the measurable capacitance (Farad) in function of the Volume of liquid (ml), and the impact of parasitic capacitance (C_{PAR}) on said resolution.
**Figure 15** is a comparative graph illustrating the capacitance resolution of an exemplary embodiment of another urine tracking device in which the shielding is omitted.

### DETAILED DESCRIPTION

The present invention will be described with respect to particular embodiments, but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

The recitation of numerical ranges by endpoints includes all numbers and fractions subsumed within the respective ranges, as well as the recited endpoints.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The terms "left," "right," "front," "back," "top," "bottom," "over," "under," and the like in the description and in the claims, if any, are used for descriptive purposes and not necessarily for describing permanent relative positions. It is to be understood that the terms so used are interchangeable under appropriate circumstances such that the embodiments described herein are, for example, capable of operation in other orientations than those illustrated or otherwise described herein. The term "coupled," as used herein, is defined as directly or indirectly connected in an electrical or nonelectrical (i.e., physical) manner Objects described herein as being "adjacent to" each other may be in physical contact with each other, in close proximity to each other, or in the same general region or area as each other, as appropriate for the context in which the phrase is used.

The term "about" is used to provide flexibility to a numerical range endpoint by providing that a given value may be "a little above" or "a little below" the endpoint. Unless otherwise stated, use of the term "about" in accordance with a specific number or numerical range should also be understood to provide support for such numerical terms or range without the term "about". For example, for the sake of convenience and brevity, when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The term "substantially" refers to the complete or nearly complete extent or degree of an action, characteristic, property, state, structure, item, or result. For example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result. For example, a composition that is "substantially free of" particles would either completely lack particles, or so nearly completely lack particles that the effect would be the same as if it completely lacked particles. In other words, a composition that is "substantially free of" an ingredient or element may still actually contain such item as long as there is no measurable effect thereof.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment. Occurrences of the phrase "in one embodiment," or "in one aspect," herein do not necessarily all refer to the same embodiment or aspect. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims and description, any of the claimed or described embodiments can be used in any combination.

In addition, it should be understood that embodiments of the invention may include hardware, software, and electronic components or modules that, for purposes of discussion, may be illustrated and described as if the majority of the components were implemented solely in hardware or software. However, one of ordinary skill in the art, and based on a reading of this detailed description, would recognize that, in at least one embodiment, the electronic based aspects of the invention may be implemented in software (e.g., instructions stored on non-transitory computer-readable medium) executable by one or more processing units, such as a microprocessor and/or application specific integrated circuits.

Accordingly, the herein described hardware may comprise a processing unit that is configured for executing the herein presented methods as software. Embodiments of the methods may be implemented in code and may be stored on a storage medium having stored thereon instructions which can be used to program a system to perform the instructions. For purposes of the present disclosure, the terms "code" or "program" cover a broad range of components and constructs, including applications, drivers, processes, routines, methods, modules, and subprograms. The terms "code" or "program" may thus be used to refer to any collection of instructions which, when executed by a processing system, performs a desired operation or operations. Additionally, alternative embodiments may include processes that use fewer than all of the disclosed operations, processes that use additional operations, processes that use the same operations in a different sequence, and processes in which the individual operations disclosed herein are combined, subdivided, or otherwise altered. Those skilled in the art can appreciate the numerous modifications and variations thereon.

To provide a solution to the above-described problems in the art, the present disclosure describes a device for tracking the daily urine output of a subject. The subject may be a human, such as a healthy human or a patient suffering from an abnormal health condition. The device is in a preferred embodiment assigned to a single subject, as will become clear from embodiments described below, but if so desired, can be repurposed for use by another subject.

The device's primary configuration is the tracking of the subject's urine output as preferably timestamped urine volume data, which may then optionally be analysed by a user. One of ordinary skill in the art understands that "daily" is used illustratively and that the timestamped urine volume data is by no means limited to a specific timeframe. Accordingly, the urine output may be tracked by the second, minute, hour, day, week, year, and so on. Moreover, as will become clear from embodiments described below, the device may also be configured for determining other urine related parameters, such as urine flow rate. The user may be the subject, or it may be another person in care of the subject, such as a healthcare professional. The device is in a preferred embodiment intended for private use at home, but if so desired, can also be used in a clinical setting, such as a hospital, rehabilitation centre, or a nursing home.

An initial overview of various components of the device is provided below and specific embodiments are then described in further detail. This initial overview is intended to aid readers in understanding the technological concepts more quickly, but is not intended to identify key or essential features thereof, nor is it intended to limit the scope of the present subject-matter.

In addition, one of ordinary skill in the art understands, and based on a reading of this detailed description, would recognize that that the various aspects can be combined unless otherwise stated. As such, any specific embodiment of a specific aspect may be understood to constitute a specific embodiment of another aspect without the explicit discussion thereof. For example, an embodiment for the configuration of the device also forms an embodiment for the manufacture of said device, an embodiment for the use of said device, and so on.

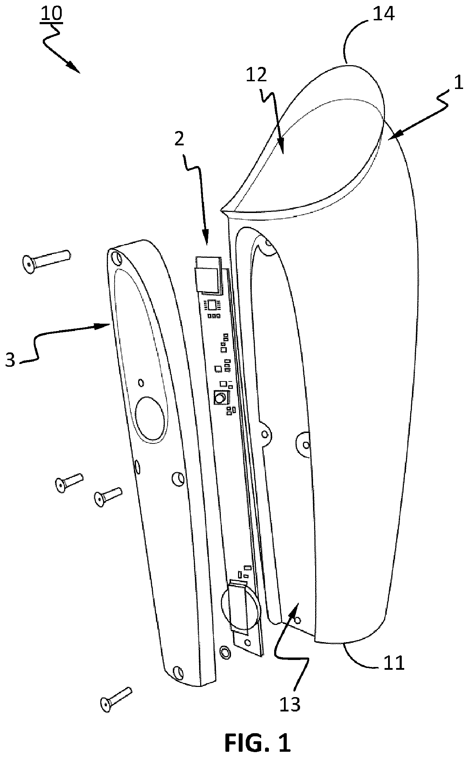

**Figure** 1 is an exploded view of a possible embodiment of the device 10 comprising an open-ended receptacle 1 for the filling of a fluid, said fluid being the subject's urine. The receptacle 1 has a bottom surface 11 and sidewalls defining an open-ended inner volume for the filling of the fluid, said open end 12 being a top of the receptacle. The device 10 further comprises a sensor circuit 2 configured for measuring a height of fluid filled into said receptacle 1. In the illustrated embodiment the electronic components of the sensor circuit 2 may be arranged on a printed circuit board (PCB) as is known in the art. The device 10 may also comprise a processing unit 5 configured for determining, *inter alia,* urine volume from data provided by the sensor circuit 2. The PCB can be fit into a recessed section 13 provided along a sidewall of said receptacle 1. Further, a cover 3 can be placed over said sensor circuit 2 to fasten and optionally seal it to the receptacle 1. This cover 3 can be fixed to the receptacle 1 with fastening elements such as screws that fit into matching screw holes provided in the receptacle 1, sensor circuit 2 and cover 3.

The sensor circuit 2 of the present device is a capacitive surface sensor circuit, i.e., a sensor circuit configured for measuring a fluid with capacitive surface sensing technology. Capacitive sensors are known in the art as devices that apply the principle of capacitive coupling to detect and measure the presence of a material, in the present case a fluid, which has a dielectric different from a set reference value. Specifically, capacitive surface sensors apply the principle of self-capacitance measuring the changes in capacitance on the surface of an electrode with respect to ground. These surface sensors may also be referred to as capacitive sensor pads. To elaborate, when applying self-capacitance sensing, the electrode functioning as a capacitor stores the available energy / capacitance in its sensing field. Advantageously, the sensor produces a larger number of field lines such that when there is liquid on the sensor surface, the available field lines might increase the amount of charge the sensor/ground capacitor can hold. Accordingly, the more available field lines, the higher the resolution of the sensor. When further considering the capacitive sensing area, the adjacent capacitive surface sensor electrodes can operate by forming a uniform electric field over the receptacle inner surface. As the container starts to fill, the rising fluid will contact a portion of one or more electrode's surface which will cause an increase in measured capacitance proportional to the amount of surface covered by the fluid.

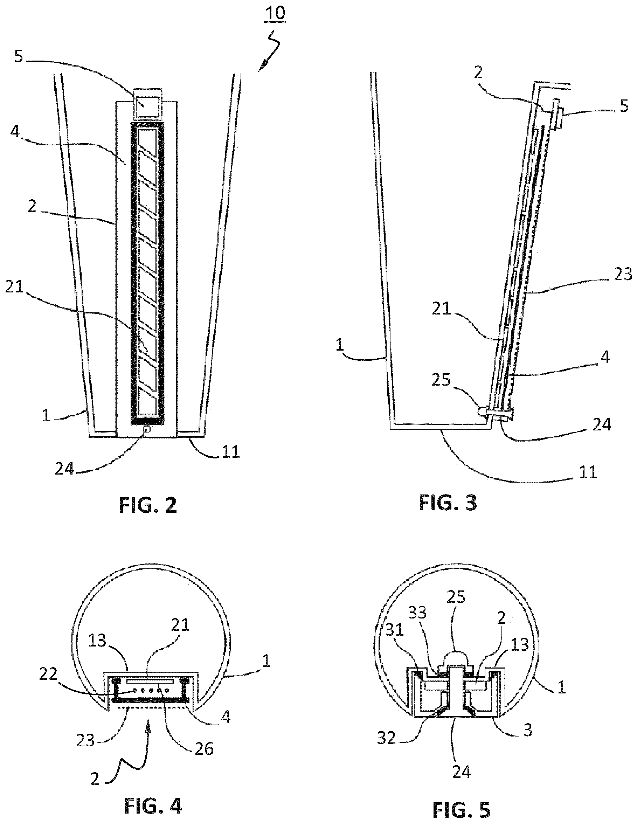

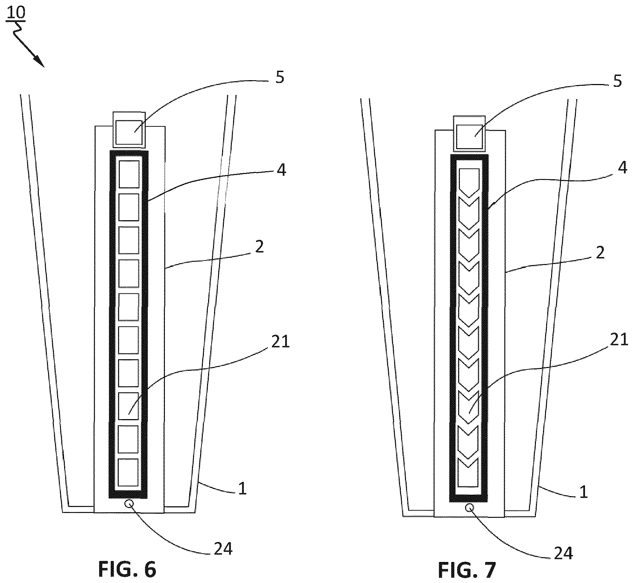

With reference to **Figure 2****,** an exemplary capacitive sensor circuit 2 is shown comprising a plurality of capacitive surface sensor electrodes 21 arranged at different distances from the bottom 11 of the receptacle 1. Other exemplary capacitive sensor circuits 2 are shown in **Figures 6** and **7****.**

Every sensor electrode 21 can be configured to detect fluid filled into the receptacle 1 at a level corresponding with the position of the sensor electrode 21. In particular, the proximity of fluid near the electrode's surface can change its capacitance which value is measured in relation to the ground (two poles of the capacitor). As such, the capacitive surface sensor electrode 21 may be configured to have its capacitance correspond with the fraction of the total electrode surface area that is in contact with fluid. The summation of the surface area of every capacitive surface sensor electrode coupled to the capacitive sensor circuit 2 will be herein referred to as the capacitive sensing area of the sensor circuit 2. Accordingly, by correlating the position of a sensor electrode 21 to its distance of said electrode 21 from the bottom 11 of the receptacle 1, the corresponding height of fluid filled into said receptacle 1 can be measured. Advantageously, the capacitive surface sensor electrodes 21 may be coupled sequentially to the capacitive sensor circuit such that the total capacitance of the capacitive sensor circuit 2 will be representative of the height of the fluid filled into the receptacle 1. Further embodiments will be described below with reference to the processing unit.

One of ordinary skill in the art may appreciate that the provision of at least two capacitive surface sensor electrodes 21 at different distances from the bottom 11 of the receptacle 1 can be sufficient to determine an initial difference in height of the fluid. For example, a first sensor electrode may be positioned at a position closer to the bottom 11 of the receptacle 1 and a second electrode may be positioned at a position closer to the top of the receptacle 1. Nonetheless, in a practical sense advantageously a greater number of sensor electrodes 21 may be provided to increase the sensor circuit's measurement accuracy. For example, **Figure 2** shows an embodiment of the capacitive sensor circuit 2 provided with ten sensor electrodes 21.

Accordingly, the total number of sensor electrodes 21 can be adjusted to the height of the receptacle 1 and the desired measurement accuracy of the capacitive sensor circuit 2. In an embodiment the capacitive sensor circuit may comprise at least three capacitive surface sensor electrodes, preferably at least four electrodes, more preferably at least five electrodes, even more preferably at least six electrodes, even more preferably at least seven electrodes, even more preferably at least eight electrodes, even more preferably at least nine electrodes, even more preferably at least ten electrodes, and so on.

Accurate capacitive sensing of a fluid like urine, however, can be difficult due to its variable dielectric properties. For example, the dielectric properties of urine vary from subject to subject, each voiding may occur from different body or room temperatures, at different flow rates, having different salt concentration, etc., each variable changing the fluid's dielectric constant and thus affecting the read-out values of the capacitive sensor circuit. Changes in dielectric properties of fluid affect capacitance measurements and therefore affect volume determination.

In some embodiments, the capacitive sensor may be grounded to the fluid filled into the receptacle. Grounding the capacitive sensor circuit to the fluid filled into the receptacle may allow for highly accurate fluid measurements to be obtained irrespective of the above-listed variables affecting the fluid's dielectric properties. To clarify, two properties of fluid like urine can severely impact on the measurement accuracy, these are the liquid potential and the conductivity. The potential of a liquid depends on many surrounding/environmental factors whereas the conductivity in the case of urine directly depends on the salt concentration. In an electrically isolated container, both properties are typically unknown at the time of measurement. Mechanically grounding the liquid therefore provides multiple beneficial effects: the potential of the liquid will be always known and equal to the battery ground, which minimizes parasitic capacitance and by closing the sensor-ground loop, the effect of variation of conductivity is greatly reduced.

With reference to **Figure 3****,** the capacitive sensor circuit 2 is shown to comprise a sensor plane 22 electrically coupled to a sequence of capacitive surface sensor electrodes 21 that are arranged towards the receptacle's inner volume and a ground plane 23 arranged on an opposite side of said capacitive sensor circuit 2. Preferably the components of the sensor circuit 2 may be arranged on a printed circuit board (PCB). For example, on a PCB the sensor plane can be a top layer and the ground plane can then be the opposing bottom layer. Advantageously, the sensor circuit's ground plane may be oriented away from the receptacle's inner volume, *i.e*., towards the outside surface, in order to improve its integration into a receptacle sidewall.

As shown in **Figure 2****,** the receptacle 1 may be provided with a recessed section 13 along one of its sidewalls with a depth that is sufficient to fully fit the capacitive sensor circuit the sensor plane and the ground plane 13 of the sensor circuit 2. Preferably, the recessed section may be disposed inwards into the receptacle, thereby reducing the receptacle's inner volume such that a continuous design may be obtained to improve the ease of holding the device 10. Alternatively, the receptacle may be provided with standing walls that form an outwards projecting section for housing the capacitive sensor circuit.

The capacitive surface sensor electrodes are disposed, preferably in a sequential manner, along at least one sidewall of the receptacle such that the capacitive sensor circuit, in particular the capacitive sensing area of the sensor circuit, extends substantially upwards from its bottom surface. This provides an efficient solution to position the capacitive surface sensor electrodes are substantially different distances receptacle at substantially different distances from the bottom surface. Advantageously, the capacitive sensing area may extend upwards in a substantially straight line along with the vertical of the receptacle or an inclination of the receptacle sidewall. An example of the latter embodiment is shown in **Figure 2****.** Alternatively, the capacitive sensing area may be bended to increase more surface along the receptacle sidewall.

The capacitive surface sensor electrodes have substantially the same surface area. The surface area as used herein refers to the surface area configured for capacitive sensing of fluid and not the total size of the electrode, which may be larger due to its electrical components. Variations in electrode surface size may cause differences in electrode read-out values depending on the variations in temperature which may be the result of changes in ambient conditions like room temperature, humidity, etc. Accordingly, by keeping the sensor electrodes at substantially the same surface size the capacitive sensor circuit 2 can be kept insensitive to variations in temperature. This may further improve the measurement accuracy of the device. An example of such an embodiment is shown in **Figure 2****.**

In an embodiment the capacitive surface sensor electrodes may be arranged such that at least a portion of the surface area of two adjacent sensor electrodes overlaps on the same distance from the bottom of the receptacle. The implementation of an exclusively sequential sensor electrode arrangement, wherein every sensor electrode is arranged at different distances from the bottom of the receptacle with no overlap, may create a sensor circuit blind spot whenever the fluid crosses from a lower-positioned lower electrode into an adjacent upper-positioned electrode. An example of such an embodiment is shown in **Figure 6****.** Accordingly, by having adjacent electrodes partially overlap a level of redundancy is provided which may further improve measurement accuracy of the device. An example of such an embodiment is shown in **Figures 2** and **7****.**

In the embodiment of **Figure 2****,** the capacitive surface sensor electrodes 21 are illustrated as a sequence of rhomboids except for the upper and lower most electrodes. Nonetheless, one of ordinary skill in the art may appreciate that any variation on the sensor electrode shape may be easily implemented. For example, the electrode shapes may include rectangles, trapezoids, arrows, and so on. For example, with reference to **Figure 6****,** in an exemplary embodiment the sensor electrode shape 21 may include squares. Further, with reference to **Figure 7****,** in an exemplary embodiment the sensor electrode shape 21 may include a sequence of downwards pointing arrows except for the upper and lower most electrodes.

In a preferred embodiment the electrode shape may have one or more parts extending downwards and/or upwards along the direction of the capacitive surface sensor electrodes to partially overlap with one or more adjacent electrodes. For example, the rhomboid-shaped electrodes in **Figure 2** allow for easy overlapping of electrode surface areas at the opposite corners of two adjacent sensor electrodes 21. Further, the arrow-shaped electrodes in **Figure 7** allow for easy overlapping of electrode surface areas in the centre of two adjacent sensor electrodes 21. Nonetheless, as shown in **Figure 2** and **7****,** the upper and lower most electrodes may have a different shape to maintain substantially the same surface area as inner sequence of electrodes. For example, the upper and lower most electrodes may have a shorter length and/or width in accordance with the sensor electrodes shapes.

As shown in **Figure 4****,** the capacitive surface sensor electrode 21 may be coupled to the sensor plane of the capacitive sensor circuit with electrical connectors 26 of substantially the same length. The electrical connector can be a lead such as copper trace for supplying a signal path between the sensor electrode 21 and the sensor circuit 2. Variations in electrical connector length may cause differences in electrode read-out values depending on variations in temperature, which may be the result of changes in ambient conditions or receptacle content, for example, if only the lower part of the receptacle warms up due the filling of warm fluid. Accordingly, by coupling the sensor electrodes with electrical connector of substantially the same length the capacitive sensor circuit 2 can be kept insensitive to any variations in temperature. This may further improve the measurement accuracy of the device.

As discussed above, the capacitive sensor circuit may be grounded to the fluid filled into the receptacle. The grounding can be achieved by providing a connection between the ground plane of the sensor circuit 2 and the receptacle's inner volume. Advantageously the grounding connection is spaced apart from the capacitive surface sensor electrodes to not influence the electrode read-out values. For example, the connection may be arranged in or near the bottom surface of the receptacle.

In an embodiment the ground plane 23 of the capacitive sensor circuit 2 may be electrically connected to the receptacle's inner volume with a conductive element 24. The filling of fluid into the receptacle 1 up to the position of said conductive element 24 can then ground the capacitive sensor circuit to said fluid. This arrangement provides an easy solution to reduce the complexity of the device 10.

As shown in **Figure 3**, according to an embodiment the conductive element 24 may be a fastening element, such as a screw or a bolt, which can be fixed into capacitive sensor circuit 2 through a screw hole provided on the body of said circuit 2, such as the PCB. Optionally, the end of said conductive element 24 may be provided with a conductive cap 25, such as a nut, configured to fix the position of said conductive element 24 and advantageously also to prevent oxidation due to frequent fluid contact. Suitable conductive materials may include metals such as copper or steel.

In an embodiment the ground plane may be cross-hatched to form a latticework of conductive material, such as copper, which is as commonly used as ground plane material in PCB. The hatched ground plane has the advantage to reduce parasitic capacitance to the capacitive surface sensor electrodes 21, thereby increasing measurement accuracy. An embodiment of a hatched ground plane 23 is shown in **Figure 4****.**

As described above, the device comprises a processing unit that is connected to the capacitive sensor circuit and configured to receive data that is representative of the height of the fluid in the receptacle from said circuit. The received fluid height data may include, for example, raw signal data, such as the voltage output of the capacitive sensor circuit wherein said voltage is representative of the height of the fluid, and/or processed signal data, such as digitally converted capacitive value, wherein said capacitive value is representative of the height of the fluid. The processing unit is then configured to determine, from the received fluid height data, a corresponding urine volume, which is defined as the total volume of the fluid filled into the receptacle.

In an embodiment, the determining of urine volume may comprise a step of converting the fluid height data into urine volume by looking up the corresponding value in a calibration curve and/or look-up table that describes the relationship between the fluid height and the receptacle's inner volume.

In a further embodiment, the processing unit may be configured to assign a timestamp to the determined urine volume. The timestamp may include information to identify the exact time and date of the voiding event. The timestamp may be provided by a time tracking device comprised in the urine tracking device. For example, the processing unit may have an internal clock. The clock may be set during manufacturing or it may synchronise when connected to a user device as will be described further below. The timestamp may be assigned to the time of voiding, defined as the time the voiding is detected by the processing unit, or to the time of measurement, defined as the time at the urine volume is determined, or both. Embodiments to detect the time of voiding will be discussed further below.

Further still, the processing unit may store the timestamped urine volume on a local memory unit, such as non-volatile memory e.g. flash drive provided in the device, or an external memory unit, such as a user device. For example, the storing of timestamped urine volume may include a storing of the timestamped urine volume in a data table. This will enable accurate tracking of a subject's urine output with every voiding session.

In an embodiment, the determining of urine volume may comprise a step of converting the fluid height data into urine volume by looking up the corresponding value in a calibration curve and/or look-up table that describes the relationship between the fluid height and the receptacle's inner volume. For example, the voltage output from the sensor circuit may be converted using a dielectric specific calibration curve and/or look-up table.

In another embodiment, the processing unit may be configured to determine a flow rate from at least two urine volumes. For example, the flow rate may be determined by subtracting the values of two consecutive urine volumes from a single voiding session and dividing by the time in between. The processing may also be configured to determine flow-rate related parameters from consecutive urine volume measurements within a single voiding session, such as the highest flow rate, average flow rate, and the like.

According to an embodiment the ancillary electronic components of the urine tracking device such as the processing unit may be housed in a separate compartment in the urine tracking device. Separating the electronic components from the capacitive sensor circuit 2 and its electrical components may further reduce the occurrence of parasitic capacitance. As shown in **Figure 3****,** these electronic components may be housed in an electronic module 5 provided for example at the top of the PCB on which the capacitive sensor circuit 2 is arranged. This allows for easier integration of the electronic components into the receptacle 1 and reduces the complexity of the device 10. The skilled person understands that the positioning of the electronic module 5 can be arranged in other parts of the device, but the top and/or bottom are advantageous for a space-saving integration.

The electronic module 5 may comprise a power source configured for suppling power to the electronic components of the device, such as the capacitive sensor circuit. The power source is preferably a battery, which is advantageously rechargeable and/or exchangeable. The device can be additionally provided with means for charging the battery. These means can consist of a port that a charger cable can plug into. Alternative provisions may include receiver coil such that the battery can be recharged wirelessly when placed on a charging pad.

As further shown in **Figure 3****,** the receptacle 1 may be provided with a recessed section 13 along one of its sidewalls with a depth that is sufficient to fully fit the electronic components of the urine tracking device 1. Preferably, this recessed section may be arranged alongside the capacitive sensor circuit and any of its components. Specifically, by integrating the electronic module 5 alongside the capacitive sensor circuit 2 a compact design can be realised which is limited to a sidewall of the device. This is particularly advantageous for a fully integrated design in which the provision of external components such as an electronic read-out unit for volume measurement (e.g. base station) is not desired.

As described above, the device comprises an electrical shielding configured to enclose the sensor plane of the capacitive sensor circuit, preferably the sensor electrodes of said sensor circuit. Electrical shielding is known in the art as providing a barrier made of a conductive material to isolate electrical devices from their surroundings. The amount of reduction is typically dependent on various factors, such as the material used and the thickness of the shielding. Suitable shielding material may include a metal such as copper or steel. Accordingly, the provision of electrical shielding may further reduce parasitic capacitance in the device.

Referring back to **Figure 2****,** the electrical shielding 4 fully surrounds the plurality of capacitive surface sensor electrodes 21, *i.e.,* from the lowermost up to the uppermost electrode. Accordingly, the electrical shielding may be arranged over the sides of the capacitive sensor circuit 2, spaced apart from the capacitive surface sensor electrodes. For example, the electrical shielding 4 may be formed by connecting a metal plate against and/or over the sides of a PCB.

As shown in **Figure 4****,** the electrical shielding 4 is disposed between the plurality of sensor electrodes 21 coupled to the sensor plane 22 and the ground plane 23. Specifically, the electrical shielding 4 is shown to be sandwiched between the sensor plane 22 and the ground plane 23. Preferably the electrical shielding 4 may be provided with a recessed section for inserting the sensor electrodes 21. This allows for the sensor electrodes 21 to be enclosed from all sides except for the recessed side, which may advantageously be oriented towards the receptacle 1 volume. Advantageously, a protective layer may be disposed over the electrodes, such as a thin wall separating the electrode surface from the inner volume. This embodiment may further reduce the parasitic capacitance in the capacitive sensor circuit 2 and protect the sensor electrodes.

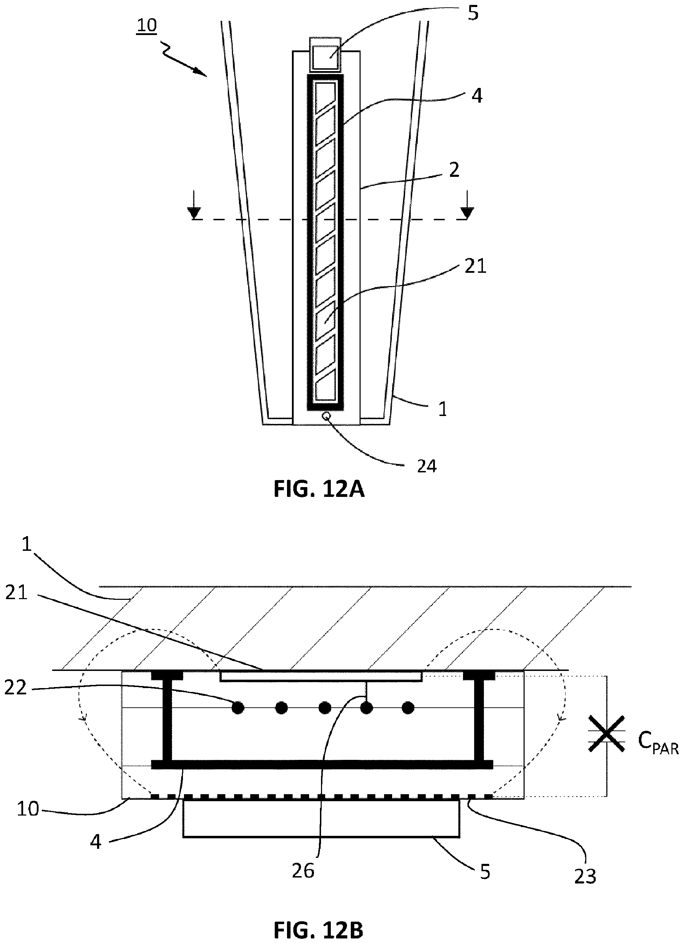

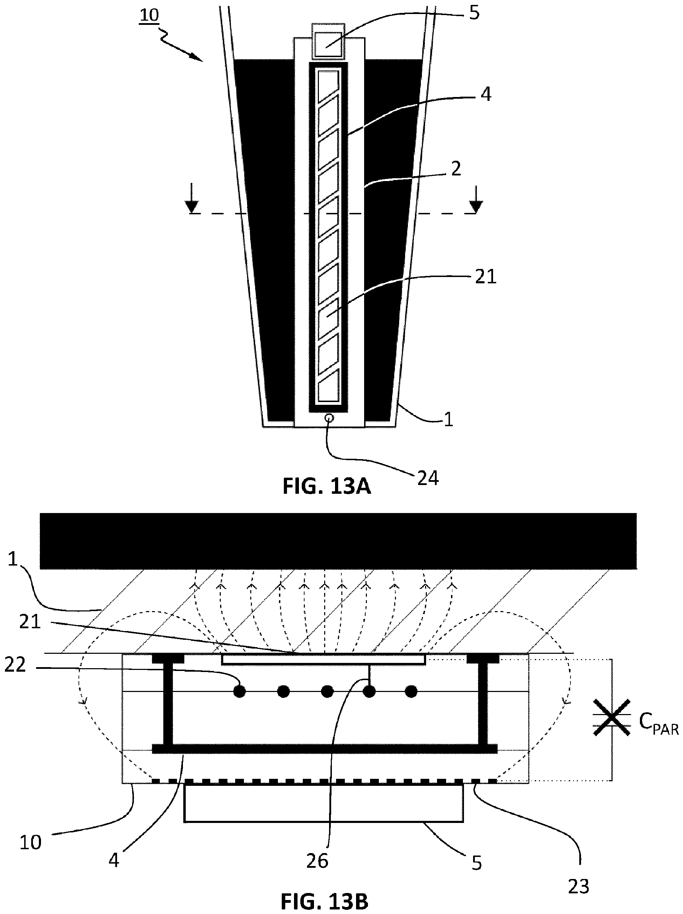

As shown in **Figures 12A-B** and **13A-B** the electrical shielding 4 is arranged between the plurality of sensor electrodes 21 coupled to the sensor plane 22 and the ground plane 23. Specifically, **Figures 12A-B** show an empty urine tracking device, i.e., without any fluid in the receptacle, and **Figures 13A-B** further show a filled urine tracking device, i.e., completely filled with fluid. The impact of the shielding 4 in the illustrated embodiment on the capacitance resolution of a urine tracking device, when empty and filled, will be discussed next.

Firstly, **Figure 12B** shows the electrical shield 4 being arranged between the plurality of sensor electrodes coupled to the sensor plane 22 and the ground plane 23 when the device is empty. This configuration allows for the plurality of sensor electrodes 21 to reduce the possible influence by parasitic capacitance (C_{PAR}) occurring between the sensor electrode 21, which is connected to the sensor plane 22, and the ground plane 23. The shielding allows for the minimisation of such parasitic capacitance (C_{PAR}), which could otherwise hinder the accuracy and resolution of the measurement. In fact, the shielding with its enclosing geometry and sandwiched placement allows for the sensor electrodes 21 and the sensor plane 22 to be shielded from the influence of occurring parasitic capacitance (C_{PAR}), effectively minimising the amount of field lines which are directed towards the ground plane 23, which could otherwise limit the capacitance measurement resolution. In comparison, the absence of the shielding would substantially increase the amount of field lines between the sensor electrode 21 and ground plane 23.

Further, **Figure 13B** shows the same urine tracking device when filled. Specifically, when focusing on the distribution of the field lines, due to the presence and arrangement of the electrical shielding 4 the field lines are distributed towards the liquid, which translates in a capacitive measurement which is less impacted by parasitic capacitance (C_{PAR}) between the plurality of sensor electrodes and the ground plane. This in turn may increase the measurement resolution of the capacitive sensing. In comparison, the absence of shielding would cause more field lines to run between the sensor electrode 21 and ground plane 23, effectively reducing the (relative) amount of field lines available for sensing of the fluid.

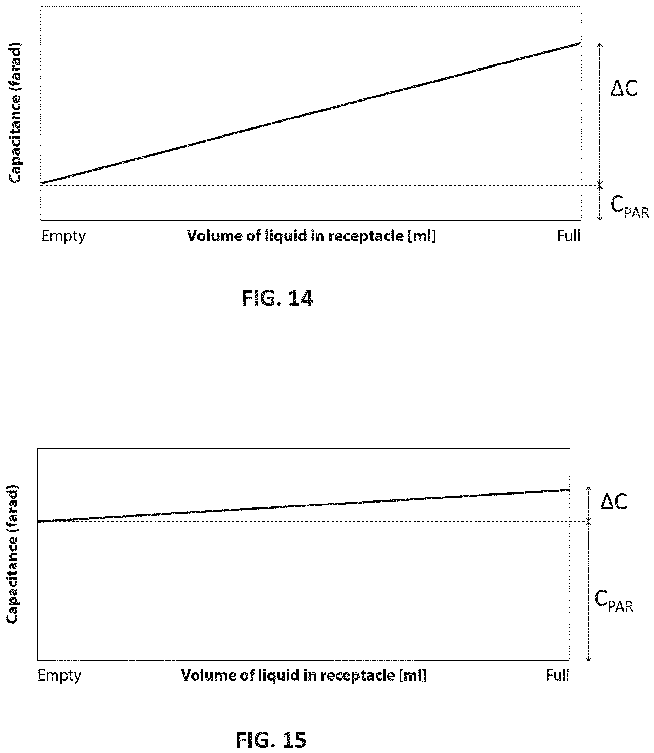

This summation of these configurations is shown in the graph of **Figure 14****,** which illustrates the capacitance resolution defined as the measurable capacitance (Farad) in function of the Volume of liquid (ml) when going from an empty state to a filled state. As can be observed, the impact of the parasitic capacitance (C_{PAR}) is minimized due to the presence of the shielding, which results in a higher resolution for the sensed capacitance (ΔC). On the other hand, the electrical shielding is omitted, the resolution of the capacitive measurement can be heavily impacted, as shown in **Figure 15** which shows a graph illustrating the capacitance resolution of a comparative exemplary embodiment of another urine tracking device in which the shielding is omitted. The absence of the shielding increases the occurrence of parasitic capacitance (C_{PAR}) which results in a significantly reduced resolution for the sensed capacitance (ΔC).

In an embodiment, the electrical shielding may be arranged between the sensor plane and the ground plane in such a way that the sensor plane and the ground plane are spaced apart by a distance corresponding to at least the thickness of said electrical shielding. In a preferred embodiment, the electrical shielding is sandwiched directly between the sensor plane and the ground plane. Such sandwich construction allows the ground plane to be in closer proximity to the sensors plane, specifically the capacitive surface sensor electrodes, enabling a more compact and space-saving design. Accordingly, the sandwich construction allows for positioning the sensor electrodes and the electronic components on the same PCB on opposite sides without influencing each other. Further, this allows designing for a compact capacitive sensor circuit limited to portion of the receptacle such as a sidewall.

In an embodiment, electrode connection traces are spaced apart from the capacitive electrodes in accordance with the formula [connection trace width] x [2]. Specifically, if the electrode connection traces are too close to the capacitive electrodes, the capacitive electrodes can influence the electrode connections, which makes the signal unusable. Accordingly, by spacing the electrode connection traces from the capacitive electrodes, the field around the electrode connection trace is not influenced by the capacitive electrode in the vicinity.

In an embodiment the electrical shielding may have a thickness of at least 0.1 mm to at most 1.0 mm.

In an embodiment the sensor plane may be spaced apart from the ground plane by at least 0.1 mm to at most 1.0 mm.

In an embodiment the electrical shielding may be spaced apart from the sensor / ground plane by at least 0.1 mm to at most 1.0 mm.

In an embodiment the capacitive electrodes and sensor plane may be spaced apart by 0.1 mm to at most 1.0 mm, at least 0.25 mm to at most 0.75 mm, preferably 0.35 mm. Specifically,

In an embodiment the shielding may be shaped to have a width which may be greater than the width of the capacitive electrodes and sensor plane width, wherein such width difference may be at least 0.5 mm to at most 2.0 mm, at least 0.75mm to at most 1.5mm, for example 1.1 mm.

The electrical shielding fully encloses the sensor plane apart from the capacitive surface sensor electrodes in such a way that their capacitive sensing area of said sensor electrodes is not obstructed by the shielding. Advantageously, the electrical shielding may be arranged over the sides of the capacitive sensor circuit, specifically of the sides of the sensor plane, spaced apart from the capacitive surface sensor electrodes. For example, the electrical shielding may cover the top, bottom and/or side walls of the sensor plane. In a preferred embodiment the electrical shielding may be formed by connecting a metal plate against and/or over the sides of a PCB. Such enclosing configuration may more effectively shield the capacitive sensing area from electromagnetic interference, which may be caused by the other electronic components of the sensor circuit 2 or externally movement, such as the user touching the receptacle with their hand. This is particularly advantageous, as it allows to lower the impact of fringing effects due to electromagnetic interference leading to parasitic ground capacitance that propagate through measurements which might hinder the accuracy of the measurements.

Referring back to **Figure** 3, when a fully integrated design of the device is contemplated wherein the capacitive sensor circuit 2 and electronic module 5 are fully fit into a sidewall of the device, the configuration of the shielding as described above may allow for the electronic module 5 (or any components thereof) to be arranged in closer proximity to said sensor circuit 2. This is particularly advantageous for a fully integrated design in which the provision of external components would typically be required to avoid the occurrence of interference affecting the read-out values. For example, by providing an external read-out unit such as a base station onto which the device is placed for volume measurement, or alternatively a bulky compartment arranged at the top / bottom of the device which needs to be electronically separated from the capacitive sensor circuit 2. Accordingly, the integrated arrangement allows for reducing the space required for the electronic components in the device, thereby making the device easier to handle while preserving more free volume which could be used for fluid collection.

In an embodiment the electrical shielding extends over the sensor plane towards receptacle's inner volume by at least 0.1 mm, preferably 0.5 mm.

In an embodiment the electrical shielding may be arranged in such a way that the directivity of the capacitive sensing area is directed towards the receptacle's inner volume. This advantageously enables the capacitive surface sensor electrodes to leverage improved directivity, as the electrical field distribution between capacitive surface sensor electrodes and the ground plane impinges only onto the sensor electrodes. This further enables the capacitive surface sensor electrodes to have improved focus on the sensing area, enabling more accurate measurements of the fluid.

In some embodiments, the capacitive sensing area angles may be at least 45° to at most 90°. This further improves the directivity of the capacitive surface sensor electrodes, and mitigates the occurrence of interference leading to parasitic capacitance, which in turn would be detrimental for the measurement accuracy.

In some embodiment the size of the electrical shielding is greater than the size of the sensor plane. This is advantageous as the positive difference in size of the electrical shield and the capacitive sensor circuit mitigates the impact of the parasitic capacitance within the operating field of the capacitive sensor. This further increases the resiliency of the sensor circuit towards electromagnetic interference as a consequence of body capacitance effects provided by the proximity (or touches) of a user.

In an embodiment the shielding may be shaped to have a width which may be greater than the width of the capacitive electrodes and sensor plane width, wherein such width difference may be at least 0.5 mm to at most 2.0 mm, at least 0.6 mm to at most 1.6mm, for example 1.1 mm. In some embodiments the electrical shielding is actively driven by a shield signal; it is an actively driven electrical shield. Preferably, the shields signal driving the actively driven electrical shield corresponds to the input signal of one or more capacitive surface sensor electrodes and/or a copy thereof, i.e., a signal equal to the capacitive surface sensor electrodes input signal, such that the capacitive sensor circuit is surrounded by its own input signal. Advantageously, this allows the capacitive sensor circuit to further mitigate environmental interferences, improving the sensitivity of the capacitive sensor circuit, as well as improving the dynamic range of the measured capacitance. This further allows to perform capacitive sensing minimizing the influence of stray interference within the operating field of the capacitive sensor. Furthermore, this enables the electrical shielding to be placed in closer proximity of the capacitive surface sensor electrodes, enabling a more compact components' integration as well as improved electromagnetic interference (EMI) rejection.

In some embodiments the device comprising an electrical shield is further configured to be an active electrical shielding by feeding said electrical shielding with the capacitive surface sensor electrodes outof-phase input signal. This advantageously enables said capacitive sensor circuit to further improve resiliency towards fringing effects as a consequence of parasitic capacitance build-up, which as a consequence leads to changes in the liquid level compromising the accuracy and reliability of the device. Referring back to **Figure 4****,** when an actively driven shield 4 is implemented in combination with the shown sandwich structure of the shield 4 between the sensor plane 22 and the ground plane 23, the electrical components can be arranged on separate sides of the capacitive sensors 21 without influencing each other, enabling the integrated structure. Specifically, the shield 4 being actively driven by a shield signal reduces the load which makes it possible to place it in closer proximity to the sensing electrode, i.e., reducing the distance between the shield 4 and the capacitive sensors 21, thus allowing for the sandwiched construction. By reducing the distance the overall thickness of the capacitive sensor circuit 2 can be reduced such that it be fully integrated into a sidewall of the device without taking excessive space from the receptacle's inner volume, instead of requiring a dedicated or connectable measurement unit, for example arranged at the top or bottom of the receptacle.

As described above, the receptacle of the present invention may be any object configured for filling and holding of urine, such as a cup, vessel, container, and the like. Accordingly, the receptacle can be made from any material that is suitable for holding urine, such as plastic. Nonetheless, the receptacle may be provided in various shapes and sizes according to the subject's preference. Exemplary embodiments of the receptacle and preferred features thereof will be discussed below.

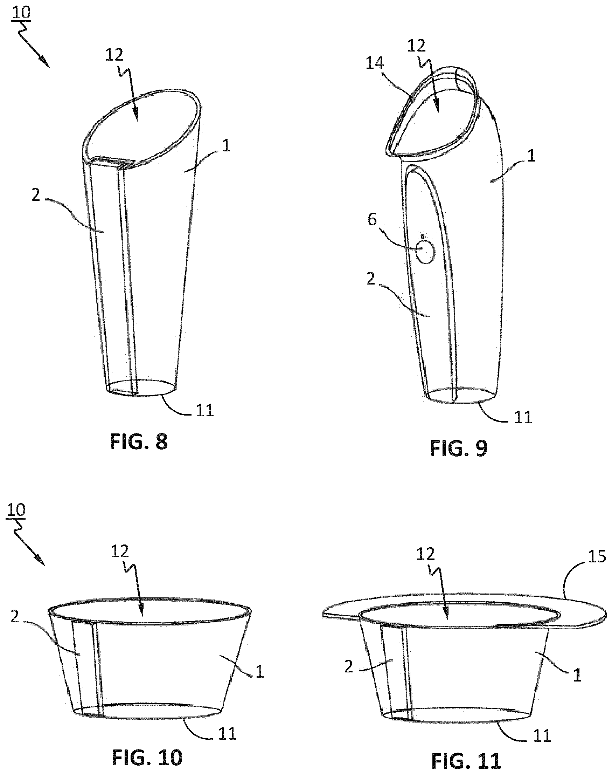

**Figure 8** is an embodiment of a handheld receptacle 1 having a longitudinal body that allows easy carrying and holding with a single free hand during voiding. Nonetheless, accidental fluid splatter may occur which is regarded as undesired by the subject.

**Figure 9** is another embodiment of a handheld receptacle 1 that is provided with a funnel 14 at the receptacle's open end 12, which is configured to facilitate voiding into the receptacle volume. The funnel 14 may also be provided in various shapes and sizes depending on the sex and comfort of the subject. The oval-shaped funnel 14 illustrated in **Figure** 9 was found to be particularly suitable for unisex purposes. Nonetheless, a longer or wider funnel 14 may also be provided to accommodate the subject's personal anatomy.

**Figure 10** is an embodiment of a placeable receptacle 1 having a bowl-shaped body that can be placed on top of a surface such as the bathroom floor or a toilet top. Advantageously the open end 12 has a large diameter to facilitate voiding into the receptacle volume.

**Figure 11** is another embodiment of a placeable receptacle 1 that is provided with a flanged rim 15 arranged along the receptacle's open end 12. This receptacle may be placed into a toilet with the rim 15 resting on top of the toilet bowl to secure the receptacle 1 in a suspended manner inside the upper portion of the toilet bowl.

The receptacle is preferably reusable and therefore benefits from having features that facilitate cleaning and disinfecting of the receptacle interior. For example, the receptacle can be provided with a layer to resist sedimentation of urine salts or supress bacterial growth. As shown in **Figure 2****,** the receptacle may be partially or fully transparent to allow the subject to detect the presence of a fluid within the receptacle. In an embodiment the receptacle's inner volume may be partially tapered towards the bottom surface. This improves the measurement resolution for smaller urine volume by letting the fluid height rise faster at the start of the voiding session. For example, the lower half of the receptacle may be partially tapered, or the lower third of the receptacle, or the lower quarter of the receptacle, and so on.

In a preferred embodiment the entire receptacle's inner volume may be tapered, i.e. from the open top down to the bottom surface, such that the same measurement resolution is maintained across all urine volumes. To clarify, a percentage sensing error would commonly result in a variable resolution across different urine volumes; for example, for a 1% sensing error would correspond with ±1ml at 100ml volume, ±2ml at 100ml volume, and so on. However, by tapering the receptacle's inner volume the percentage sensing error can be correlated to a constant volume sensing error; for example, ±5ml at 100ml volume, ±5ml at 200ml volume, and so on. The above-discussed embodiments of **Figures 8** to **11** illustrate fully tapered receptacles.

As discussed above, the device 10 may comprise a cover 3 that can be fastened to the receptacle to safely secure the sensor circuit 2 and/or any electronic components of the device 10 to the receptacle 1. The cover 3 can be fixed to the receptacle 1 with fastening elements, such as screws that fit into matching screw holes provided in the receptacle 1 and cover 3. Alternatively, the cover 3 can also be fastened using other fastening elements known in the art, such as a click-on mechanism, hooks, etc., and/or fixed using a fixing material such as glue or a metal in a welding process.

In an embodiment the device may comprise one or more sealing elements configured to seal the device such that fluid cannot reach the capacitive sensor circuit and/or any electronic components. The sealing element may be a compressible plastic, such as silicone, thermoplastic elastomers, rubber, and/or any other sealing element known in the art. Advantageously the sealing element may be integrated into the receptacle and/or the cover so that it can more easily and tightly sealed when fixing the cover.

With reference to **Figure 5****,** an exemplary arrangement of sealing elements is described that is aimed to waterproof the device 10. In particular, a first sealing element 31 may be provided in the front and/or sides of the cover 3, when placed into a recessed section of the receptacle 13, so that it prevents any penetration of fluid through the border formed between the cover 3 and the receptacle 1. Further, a second sealing element 32 may be provided between the cover 3 and the conductive element 24, preferably around an opening provided in the cover 3 for placement of said conductive element 24, so that it prevents any penetration of fluid, such as water, through said opening in the cover 3. Further still, a third sealing element 33 may be provided between the receptacle 1 and the conductive cap 25, preferably around an opening provided in the receptacle 1 for placement of the conductive element 24, such that it prevents any penetration of fluid through said opening in the receptacle 1. The advantage of the described embodiment provides a fully waterproof device 10 that is suitable for in-toilet use and can also be safely cleaned by submerging the device 10 in clean water.

In an embodiment the urine tracking device may comprise a reference sensor configured to detect the presence and/or absence of fluid in the receptacle. The capacitive sensor circuit as described above may have difficulty distinguishing between a completely full receptacle, whereby the capacitive sensing area of the sensor circuit is completely covered with fluid, and a completely empty receptacle, whereby none of the capacitive sensing area of the sensor circuit is contact with fluid, since both the states may result in a lack of variation between the different capacitive surface sensor electrodes of the capacitive sensor circuit when it is grounded to said fluid. As such, the provision of an additional reference sensor, separate from the sensor circuit, may provide a reliable solution for distinguishing between the receptacle's empty or full state.

The reference sensor may in principle comprise any sensor type that is capable of directly or indirectly detecting the presence of fluid in the receptacle. Exemplary embodiments of the reference sensor are provided: In an embodiment the reference sensor may comprise a temperature sensor configured to detect a change in receptacle temperature due to the warmth of the fluid. In an embodiment the reference sensor may comprise a pressure sensor configured to detect the weight of the fluid; for example, disposed on the bottom surface of the receptacle. In an embodiment the reference sensor may comprise an optical sensor configured to detect reflection of light from the fluid surface.

In a further embodiment, the reference sensor may be connected to the processing unit and said processing unit may be configured to receive data representative of the presence of fluid in the receptacle from said reference sensor and control the operation of the urine tracking device based on measurements made by said reference sensor. The received fluid stability data may include raw signal data, such as the voltage output of the reference sensor, and/or processed data, such as a digitally converted output of the reference sensor, such that the processing unit can determine one or more values from said data that are representative of the presence of the fluid in the receptacle.

In a preferred embodiment, the reference sensor may comprise a reference capacitive sensor electrode that is configured to provide a baseline value that is independent of the presence of a fluid in the receptacle. In particular, the reference capacitive electrode is arranged such that its capacitance value is not changed when fluid is filled into the receptacle. Advantageously, the reference capacitive sensor electrode may be disposed between the capacitive surface sensor electrodes and the electrical shielding, so that it is sufficiently separated from the fluid and also benefits from a reduction in parasitic capacitance. This arrangement provides an easy solution to reduce the complexity of the device, since the reference capacitive sensor electrode can be more easily connected to the processing unit without the increased complexity of any of the above-described exemplary sensor components.

In a further preferred embodiment, the reference capacitive sensor electrode may be connected to the processing unit and said processing unit may be configured to receive a baseline value from said reference capacitive electrode, such that the processing unit can determine the presence and/or absence of fluid in the receptacle by comparing said baseline value with the capacitance values measured by one or more capacitive surface sensor electrodes of the capacitive sensor circuit.

In an exemplary the processing unit may determine the presence of the fluid by subtracting the value measured by the lowermost capacitive sensor from the reference baseline value to obtain a capacitance difference representative of the presence or absence of fluid in the receptacle. In a first example, if the capacitive sensor reads 1000 and the baseline value is also 1000 the difference will be 0, which corresponds with the absence of fluid in the receptacle. In a further example, if the capacitive sensor reads 2000 and the baseline value is 1000 the difference will be 1000, which may correspond with the presence of fluid in the receptacle. Advantageously, the processing unit may be configured to compare said capacitance difference with a pre-set threshold value, whereby the presence or absence of fluid is determined when the capacitance difference surpasses said set threshold value.

In an embodiment the processing unit may be configured to initiate the fluid height measurement by the capacitive sensor circuit when the reference sensor detects the presence of fluid in the receptacle. For example, the processing unit may reduce power to the capacitive sensor circuit when no fluid is in the receptacle to reduce power consumption and improve battery life. For example, the processing unit may initiate the sampling time for determining a urine flow rate as soon as fluid is detected in the receptacle. In a preferred embodiment the fluid height measurement may be initiated when the above-described capacitance difference surpasses said a pre-set threshold value.

In an embodiment the processing unit may be configured to verify correct operation of the capacitive sensor circuit by comparing the sensor circuit response time with data from the reference sensor. For example, any mismatch between the sensor circuit and the reference sensor may be an indication that a sensor electrode is malfunctioning or alternatively that the receptacle may need to be cleaned due to built-up sediment.

In an embodiment the urine tracking device may comprise a fluid stability sensor configured for determining the stability of the fluid within receptacle. Excessive tilting or shaking of the receptacle may disturb the level of the fluid and cause the fluid height data measured by the sensor circuit not to correspond with the correct values of the urine volume. Accordingly, by providing the device with a sensor to detect receptacle tilt and/or shaking the recording of incorrect urine volume values can be avoided.

In a preferred embodiment the fluid stability sensor may comprise a tilt sensor, which is known in the art as sensor type that is capable of directly or indirectly measuring the tilt of the receptacle. Particularly suitable sensors may include an accelerometer, a gyroscope, an inclinometer, or the like. Advantageously the tilt sensor may be configured to determine the tilt angle and/or direction relative to a reference plane, such as the receptacle's vertical. More advantageously the tilt sensor may be configured to track the receptacle movement with six degrees of freedom, including the pitch, roll and yaw.

In a preferred embodiment the fluid stability sensor may comprise a force sensor, which is known in the art as sensor type that is capable of directly or indirectly detecting the movement of the receptacle. Particularly suitable sensors may include an accelerometer, a vibration sensor, or the like. Advantageously the force sensor may be configured to detect both force magnitude and direction. In a particular embodiment the force sensor may also function as the above-described tilt sensor.

In a further embodiment, the fluid stability sensor may be connected to the processing unit and said processing unit may be configured to receive data that is representative of the stability of the fluid in the receptacle from said fluid stability sensor and control the operation of the urine tracking device based on measurements made by said fluid stability sensor. The received fluid stability data may include raw signal data, such as the voltage output of the fluid stability sensor, and/or processed data, such as a digitally converted output of the fluid stability sensor, so that the processing unit may determine one or more values from said data that are representative of the stability of the fluid in the receptacle.

In an embodiment the processing unit may be configured to correct the fluid height data when excessive fluid instability is detected. For example, the processing unit may repeat the fluid height measurement by disregarding the incorrect fluid height data and receiving new corrected data when the fluid has sufficiently stabilised. Alternatively, the processing unit may altogether delay measurement by the sensor circuit until the fluid is sufficiently stabilised.

In an embodiment the processing unit may be configured to provide user-feedback based when excessive fluid instability is detected. For example, the device may alert the subject that the fluid is too unstable to reliable determine the urine volume and request, either directly with a message or indirectly with an audio or visual cue, that the subject should stabilise the receptacle for measurement.

In an embodiment the processing unit may be configured to correct the urine volume according to the fluid instability data, in particular by correcting the fluid height data to the receptacle tilt. The correcting of urine volume may include a step of determining the tilt angle and/or direction, preferably with reference to the receptacle's vertical, and converting the fluid height data into corrected fluid height data by looking up the corresponding value in a calibration curve and/or look-up table that describes the relationship between the fluid height and the receptacle's tilt angle and/or direction. Accordingly, the corrected fluid height data may then be used to determine the correct urine volume. This embodiment may allow for measurement on an unstable or moving surface, such as the subject's hand.

In an embodiment the urine tracking device may comprise a user interface module configured for subject's interaction with the device. For example, the user interface module may be a button or set of buttons that the subject can press or otherwise interact with to operate the urine tracking device. The processing unit in turn may be configured to receive the subject's input from the user interface module and control the operation of the urine tracking device based on said subject's input received from the user interface module.

In an embodiment the processing unit may be configured to initiate the fluid height measurement by the capacitive sensor circuit when the subject interacts with the user interface module. This may reduce power to the capacitive sensor circuit between measurement to reduce power consumption and improve battery life.

In an embodiment the processing unit may be configured to convey one or more instructions to the subject regarding the use of the urine tracking device. For example, with reference to the above embodiments the processing unit may indicate that the receptacle is tilted and/or the fluid is too turbulent to enable a correct measurement. The instructions may be conveyed either directly with a message or indirectly with an audio or visual cue.

According to an embodiment the user interface module may be disposed in the cover of the device. As shown in **Figure 9****,** the user interface module 6 may integrated into the cover 3 housing the capacitive sensor circuit 2 and/or the electronic module 5. This allows for easier integration of the user interface module 6 into the receptacle 1 and reduces the complexity of the device 10.

In an embodiment the urine tracking device may be configured for connecting to a personal computing device, such as a user smartphone or personal computer, and transmitting subject data to said output device via a data link. The communication module may also be configured to directly transmit the subject data to a remote computing device, such as a server, or, alternatively, indirectly through the personal computing device. Advantageously the communication module is comprised in the above-discussed electronic 5 to realise an integrated design.

The data link may be a wired or wireless connection, advantageously established by integrating a communication module within the urine tracking device. The communication module may be configured to establish short-range communications, for example BLE or Wi-Fi, and/or long-range communications, for example LTE or cellular. Providing the device with a long-range communication module has the added benefit of tracking subject data in an environment with limited internet access, or alternatively increase the subject's comfort during movement of the subject, such as for work or travels.

Accordingly, it should be noted that a plurality of hardware and software-based devices, as well as a plurality of different structural components may be utilized to implement the invention. For example, "server" and "computing device" described in the specification can include one or more processing units, one or more computer-readable medium modules, one or more input/output interfaces, and various connections (*e.g*., a system bus) connecting the components.

The computing devices may be provided with analytical software, such as an application, which is configured to analyse the received data and optionally determine various features from said data. In turn, the remote computing devices may be configured to analyse the received data using more complicated algorithms, for example, by means of machine learning model.

The computing devices may comprise a storage means for storing both received and analysed data. Further, the computing device may comprise an output device, such as a display, for displaying the received and analysed data to the subject or a user of the device, for example a healthcare professional. The data may be presented in the form of, for example, tables, charts and/or timelines to improve the conveying of information to the subject or user.

In an embodiment the computing device may be configured to determine a subject specific profile which may be used, for example, to evaluate one or more urological functions of the subject. The subject specific profile may be used by the subject or user to detect any abnormal health conditions. Also, the subject specific profile may be used to provide user feedback to promote lifestyle quality improvements in the form of, for example, information about lack of hydration.

In an embodiment the urine tracking device may comprise a subject identification module. The identification module may include information for identifying the subject's identity. For example, the identification module may be an integrated circuit that stores a unique identifier that has been assigned to the device and/or the subject, and optionally any complementary authenticator, such as an authentication key. The subject identification module may be used when connecting the urine tracking device to a computing device according to an embodiment as described herein.

Accordingly, in a preferred embodiment the urine tracking device may be a single subject urine tracking device. The provision of a subject identification module may aid in tracking the correct subject data on a computing device and hence optimise the functionality of the device. This may be particularly beneficial in an embodiment wherein multiple urine tracking devices connect to the same computing device, such as a remote server and/or a clinical setting. For example, the subject identification module may aid in the tracking of data by a healthcare professional and ensure no mistakes can occur due to mixing-up of data.

## Claims

1. Device (10) for tracking a subject's daily urine output comprising
- a receptacle (1) having a bottom surface (11) and sidewalls defining an open-ended inner volume for the filling of a fluid, said open end (12) being a top of the receptacle (1);
- a capacitive sensor circuit (2) configured for measuring the height of the fluid filled into the receptacle (1) within a capacitive sensing area;
said sensor circuit (2) comprising a sensor plane (22) forming at least one first layer within the sensor circuit (2), at least two capacitive surface electrodes (21), a plurality of electrical connectors (26) that couple the capacitive surface sensor electrodes (21) to the sensor plane (22), and a ground plane (23) forming at least one second layer within the sensor circuit (2); wherein the capacitive surface electrodes (21) have substantially the same surface area and are arranged along at least one sidewall of the receptacle (1) at substantially different distances from the bottom surface (11) such that the capacitive sensing area extends substantially upwards from the bottom surface (11); and
- an electronic module (5) comprising a processing unit connected to the capacitive sensor circuit (2) which is configured to:
- receive fluid height data from the capacitive sensor circuit (2)
- determine, from said fluid height data, a corresponding urine volume
- optionally assign a timestamp to said urine volume; and
- store said, optionally timestamped, urine volume on a memory unit;
- **characterised in that** the device (10) further comprises an electrical shielding (4) sandwiched between the sensor plane (22) and the ground plane (23); wherein the electrical shielding (4) fully encloses the sensor plane (22) apart from the capacitive surface sensor electrodes (21) such that it shields the capacitive sensing area from interference and minimizes parasitic capacitance between the sensor plane (22) and the ground plane (23).

2. The device (10) according to claim 1 wherein the capacitive sensor (2) is grounded to the fluid filled into the receptacle (1).

3. The device (10) according to one of the preceding claims wherein the capacitive sensor circuit (2) comprises a conductive element connecting the receptacle's inner volume to the ground plane (23); preferably wherein the conductive element comprises a conductive fastening element (24), such as a bolt, provided with a conductive cap (25).

4. The device (10) according to one of the preceding claims wherein the capacitive surface sensor electrodes (21) are arranged such that at least a portion of the surface area of two adjacent sensor electrodes (21) overlaps on the same distance from the bottom of the receptacle (1).

5. The device (10) according to one of the preceding claims wherein the electrical shielding (4) is actively driven by a shield signal which corresponds to an input signal of the capacitive surface sensor electrodes (21) and/or a copy thereof.

6. The device (10) according to one of the preceding claims wherein the capacitive sensor circuit (2) and the electronic module (5) are integrated in at least one sidewall of the receptacle (1), preferably arranged alongside each other.

7. The device (10) according to one of the preceding claims wherein the receptacle (1) has a recessed section (13) along one of its sidewalls with a depth that is sufficient to fit the capacitive sensor circuit (2) and the electronic module (5), preferably alongside the capacitive sensor circuit (2); and wherein the device (10) comprises a cover (3) configured to secure the capacitive sensor circuit (2) within said recessed section (13); preferably wherein the device (10) further comprises one or more sealing elements (31) disposed between the receptacle (1) and the cover (3) so that fluid cannot reach the capacitive sensor circuit (2).

8. The device (10) according to one of the preceding claims wherein the receptacle's inner volume is tapered towards the bottom surface (11).

9. The device (10) according to one of the preceding claims wherein the electrical connectors (26) are of substantially the same length.

10. The device (10) according to one of the preceding claims wherein the ground plane (23) is hatched.

11. The device (10) according to one of the preceding claims wherein the processing unit is configured to determine the urine volume by looking up the corresponding value in a calibration curve and/or look-up table that describes the relationship between the fluid height and the receptacle's open-ended volume.

12. The device (10) according to one of the preceding claims wherein the capacitive sensor circuit (2) comprises a reference capacitive sensor electrode that is configured to determine a baseline value independent of the presence of a fluid in the receptacle (1); wherein the processing unit is configured to receive the baseline value from said reference capacitive electrode and determine the presence and/or absence of fluid in the receptacle (1) by comparing said baseline value with a capacitance value measured by one or more capacitive surface sensor electrodes (21); preferably wherein reference capacitive sensor electrode is disposed between the capacitive surface sensor electrodes (21) and the electrical shielding (4).

13. The device (10) according to any one of the preceding claims further comprising a fluid stability sensor configured for determining the stability of the fluid within the receptacle (1) and wherein the processing unit is configured to correct the fluid height data by delaying and/or repeating the fluid height measurement by the capacitive sensor circuit (2) when fluid instability is detected; preferably wherein the processing unit is further configured to provide user-feedback to stabilise the receptacle (1) for measurement when fluid instability is detected; preferably with an audio and/or visual cue.

14. The device (10) according to any one of the preceding claims wherein the urine tracking device (10) comprises a tilt sensor configured for measuring the tilt of the receptacle (1) and wherein the processing unit is configured to correct the fluid height data by determining the tilt angle and/or direction and converting the fluid height data into a corrected fluid height data; preferably by looking up the corrected fluid height data in a calibration curve and/or look-up table that describes the relationship between the fluid height and the receptacle's tilt angle and/or direction.

15. The device (10) according to any one of the preceding claims wherein the urine tracking device (10) comprises a communication module that is configured to connect to a computing device (10), such as a smartphone or personal computer, and sending the determined urine volume, preferably the timestamped urine volume, to said computing device (10); and wherein the urine tracking device (10) comprises a subject identification module that is configured to identify the subject when connecting the urine tracking device (10) to the computing device (10).

## Patentansprüche

1. Vorrichtung (10) zur Verfolgung einer täglichen Urinausscheidung eines Subjekts, umfassend:
- einen Behälter (1) mit einer Bodenoberfläche (11) und Seitenwänden, die ein offenendiges Innenvolumen zum Füllen mit einem Fluid aufweist, wobei das offene Ende (12) die Oberseite des Behälters (1) ist;
- eine kapazitive Sensorschaltung (2), die konfiguriert ist, um die Höhe des in den Behälter (1) gefüllten Fluids innerhalb eines kapazitiven Abfühlbereichs zu messen;
wobei die Sensorschaltung (2) eine Sensorebene (22), die mindestens eine erste Schicht innerhalb der Sensorschaltung (2) bildet, mindestens zwei kapazitive Oberflächenelektroden (21), eine Vielzahl von elektrischen Verbindern (26), welche die kapazitiven Oberflächensensorelektroden (21) an die Sensorebene (22) koppeln, und eine Erdungsebene (23) umfasst, die mindestens eine zweite Schicht innerhalb der Sensorschaltung (2) bildet; wobei die kapazitiven Oberflächenelektroden (21) im Wesentlichen denselben Oberflächenbereich haben und entlang mindestens einer Seitenwand des Behälters (1) in im Wesentlichen unterschiedlichen Abständen zu der Bodenoberfläche (11) angeordnet sind, so dass der kapazitive Abfühlbereich sich von der Bodenoberfläche (11) im Wesentlichen aufwärts erstreckt; und
- ein elektronisches Modul (5), umfassend eine Verarbeitungseinheit, die mit der kapazitiven Sensorschaltung (2) verbunden ist, konfiguriert zum:
- Empfangen von Fluidhöhendaten von der kapazitiven Sensorschaltung (2)
- Bestimmen eines entsprechenden Urinvolumens aus den Fluidhöhendaten
- gegebenenfalls Zuweisen eines Zeitstempels zu dem Urinvolumen; und
- Speichern des gegebenenfalls mit Zeitstempel versehenen Urinvolumens in einer Speichereinheit;
- **dadurch gekennzeichnet, dass** die Vorrichtung (10) des Weiteren eine elektrische Abschirmung (4) umfasst, die sandwichartig zwischen der Sensorebene (22) und der Erdungsebene (23) liegt; wobei die elektrische Abschirmung (4) die Sensorebene (22) mit Ausnahme der kapazitiven Oberflächensensorelektroden (21) vollständig umschließt, so dass sie den kapazitiven Abfühlbereich vor Interferenz abschirmt und parasitäte Kapazität zwischen der Sensorebene (22) und der Erdungsebene (23) minimiert.

2. Vorrichtung (10) nach Anspruch 1, wobei der kapazitive Sensor (2) bezogen auf das in den Behälter (1) gefüllte Fluid geerdet ist.

3. Vorrichtung (10) nach einem der vorhergehenden Ansprüche, wobei die kapazitive Sensorschaltung (2) ein leitfähiges Element umfasst, welches das Innenvolumen des Behälters mit der Erdungsebene (23) verbindet; wobei vorzugsweise das leitfähige Element ein leitfähiges Befestigungselement (24) umfasst, wie einen Bolzen, der mit einer leitfähigen Kappe (25) bereitgestellet ist.

4. Vorrichtung (10) nach einem der vorhergehenden Ansprüche, wobei die kapazitiven Oberflächensensorelektroden (21) so angeordnet sind, dass mindestens ein Anteil des Oberflächenbereichs von zwei benachbarten Sensorelektroden (21) in dem gleichen Abstand zu dem Boden des Behälters (1) überlappt.

5. Vorrichtung (10) nach einem der vorhergehenden Ansprüche, wobei die elektrische Abschirmung (4) durch ein Abschirmsignal, das einem Eingabesignal der kapazitiven Oberflächensensorelektroden (21) und/oder einer Kopie davon entspricht, aktiv angesteuert wird.

6. Vorrichtung (10) nach einem der vorhergehenden Ansprüche, wobei die kapazitive Sensorschaltung (2) und das elektronische Modul (5) in mindestens eine Seitenwand des Behälters (1) integriert sind, wobei sie vorzugsweise nebeneinander angeordnet sind.

7. Vorrichtung (10) nach einem der vorhergehenden Ansprüche, wobei den Behälter (1) einen ausgesparten Abschnitt (13) entlang einer ihrer Seitenwände mit einer Tiefe aufweist, die ausreicht, um die kapazitive Sensorschaltung (2) und das elektronische Modul (5), vorzugsweise neben der kapazitiven Sensorschaltung (2), einzupassen; und wobei die Vorrichtung (10) eine Abdeckung (3) umfasst, die konfiguriert ist, um die kapazitive Sensorschaltung (2) innerhalb des ausgesparten Abschnitts (13) zu sichern; wobei vorzugsweise die Vorrichtung (10) des Weiteren ein oder mehrere Dichtungselemente (31) umfasst, das/die zwischen des Behälters (1) und der Abdeckung (3) angeordnet ist/sind, so dass Fluid die kapazitive Sensorschaltung (2) nicht erreichen kann.

8. Vorrichtung (10) nach einem der vorhergehenden Ansprüche, wobei das Innenvolumen des Behälters in Richtung der Bodenoberfläche (11) zuläuft.

9. Vorrichtung (10) nach einem der vorhergehenden Ansprüche, wobei die elektrischen Verbinder (26) im Wesentlichen dieselbe Länge haben.

10. Vorrichtung (10) nach einem der vorhergehenden Ansprüche, wobei die Erdungsebene (23) schraffiert ist.

11. Vorrichtung (10) nach einem der vorhergehenden Ansprüche, wobei die Verarbeitungseinheit konfiguriert ist, um das Urinvolumen zu bestimmen, indem der entsprechende Wert in einer Kalibrierkurve und/oder Nachschlagtabelle nachgeschlagen wird, die die Beziehung zwischen der Fluidhöhe und dem offenendigen Volumen des Behälters beschreibt/beschreiben.

12. Vorrichtung (10) nach einem der vorhergehenden Ansprüche, wobei die kapazitive Sensorschaltung (2) eine kapazitive Referenzsensorelektrode umfasst, die konfiguriert ist, um unabhängig von der Anwesenheit eines Fluids in des Behälters (1) einen Basislinienwert zu bestimmen; wobei die Verarbeitungseinheit konfiguriert ist, um den Basislinienwert von der kapazitiven Referenzelektrode zu empfangen und die Anwesenheit und/oder Abwesenheit von Fluid in des Behälters (1) zu bestimmen, indem der Basislinienwert mit einem Kapazitätswert verglichen wird, der durch eine oder mehrere kapazitive Oberflächensensorelektroden (21) gemessen wird; wobei vorzugsweise die kapazitive Referenzsensorelektrode zwischen den kapazitiven Oberflächensensorelektroden (21) und der elektrischen Abschirmung (4) angeordnet ist.

13. Vorrichtung (10) nach einem der vorhergehenden Ansprüche, des Weiteren umfassend einen Fluidstabilitätssensor, der konfiguriert ist, um die Stabilität des Fluids innerhalb des Behälters (1) zu bestimmen, und wobei die Verarbeitungseinheit konfiguriert ist, um die Fluidhöhendaten durch Verzögern und/oder Wiederholen der Fluidhöhenmessung mittels der kapazitiven Sensorschaltung (2) zu korrigieren, wenn Fluidinstabilität detektiert wird; wobei die Verarbeitungseinheit vorzugsweise des Weiteren konfiguriert ist, um Benutzerrückmeldung bereitzustellen, um den Behälter (1) zur Messung zu stabilisieren, wenn Fluidinstabilität detektiert wird; vorzugsweise mit einem hörbaren und/oder visuellen Hinweis.

14. Vorrichtung (10) nach einem der vorhergehenden Ansprüche, wobei die Urinverfolgungsvorrichtung (10) einen Kippsensor umfasst, der konfiguriert ist, um die Kippung des Behälters (1) zu messen, und wobei die Verarbeitungseinheit konfiguriert ist, um die Fluidhöhendaten zu korrigieren, indem der Kippwinkel und/oder die Kipprichtung bestimmt wird/werden und die Fluidhöhendaten in korrigierte Fluidhöhendaten konvertiert werden; vorzugsweise durch Nachschlagen der korrigierten Fluidhöhendaten in einer Kalibrierkurve und/oder Nachschlagtabelle, welche die Beziehung zwischen der Fluidhöhe und dem Kippwinkel und/oder der Kipprichtung des Behälters beschreibt/beschreiben.

15. Vorrichtung (10) nach einem der vorhergehenden Ansprüche, wobei die Urinverfolgungsvorrichtung (10) ein Kommunikationsmodul umfasst, das konfiguriert ist, um mit einer Rechenvorrichtung (10), wie einem Smartphone oder Personal Computer, verbunden zu werden und das bestimmte Urinvolumen, vorzugsweise das mit Zeitstempel versehene Urinvolumen, an die Rechenvorrichtung (10) zu senden; und wobei die Urinverfolgungsvorrichtung (10) ein Subjektidentifikationsmodul umfasst, das konfiguriert ist, um das Subjekt zu identifizieren, wenn die Urinverfolgungsvorrichtung (10) mit der Rechenvorrichtung (10) verbunden wird.

## Revendications

1. Dispositif (10) de suivi du débit quotidienne d'urine d'un sujet comprenant :
- un récipient (1) ayant une surface inférieure (11) et des parois latérales définissant un volume intérieur qui termine ouvert pour le remplissage d'un fluide, ladite extrémité qui termine ouverte (12) étant une partie supérieure du récipient (1) ;
- un circuit de capteur capacitif (2) configuré pour mesurer la hauteur du fluide remplissant le récipient (1) à l'intérieur d'une zone de détection capacitive ;
ledit circuit de capteur (2) comprenant un plan de capteur (22) formant au moins une première couche dans le circuit de capteur (2), au moins deux électrodes de surface capacitives (21), une pluralité de connecteurs électriques (26) qui couplent les électrodes de capteur de surface capacitives (21) au plan de capteur (22), et un plan de masse (23) formant au moins une deuxième couche dans le circuit de capteur (2) ; les électrodes de surface capacitives (21) ayant sensiblement la même zone de surface et étant agencées le long d'au moins une paroi latérale du récipient (1) à des distances sensiblement différentes de la surface inférieure (11), de telle sorte que la zone de détection capacitive s'étend sensiblement vers le haut à partir de la surface inférieure (11) ; et
- un module électronique (5) comprenant une unité de traitement connectée au circuit de capteur capacitif (2) qui est configuré pour :
- recevoir des données de hauteur de fluide du circuit de capteur capacitif (2)
- déterminer, à partir desdites données de hauteur de fluide, un volume d'urine correspondant
- attribuer éventuellement un horodatage audit volume d'urine ; et
- stocker ledit volume d'urine, éventuellement horodaté, sur une unité de mémoire ;
- **caractérisé en ce que** le dispositif (10) comprend en outre un blindage électrique (4) pris en sandwich entre le plan de capteur (22) et le plan de masse (23) ; le blindage électrique (4) entourant entièrement le plan de capteur (22) à l'écart des électrodes de capteur de surface capacitives (21) de manière à blinder la zone de détection capacitive des interférences et à réduire au minimum la capacité parasite entre le plan de capteur (22) et le plan de masse (23).

2. Dispositif (10) selon la revendication 1, le capteur capacitif (2) étant mis à la masse avec le fluide remplissant le réceptacle (1).

3. Dispositif (10) selon l'une des revendications précédentes, le circuit de capteur capacitif (2) comprenant un élément conducteur reliant le volume intérieur du récipient au plan de masse (23) ; de préférence l'élément conducteur comprenant un élément de fixation conducteur (24), tel qu'un boulon, muni d'un capuchon conducteur (25).

4. Dispositif (10) selon l'une des revendications précédentes, les électrodes de capteur de surface capacitives (21) étant disposées de telle sorte qu'au moins une partie de la zone de surface de deux électrodes adjacentes (21) du capteur se chevauche à la même distance du fond du récipient (1).

5. Dispositif (10) selon l'une des revendications précédentes, le blindage électrique (4) étant activement piloté par un signal de blindage qui correspond à un signal d'entrée des électrodes de capteur de surface capacitives (21) et/ou à une copie de celles-ci.

6. Dispositif (10) selon l'une des revendications précédentes, le circuit de capteur capacitif (2) et le module électronique (5) étant intégrés dans au moins une paroi latérale du récipient (1), de préférence agencés l'un à côté de l'autre.

7. Dispositif (10) selon l'une des revendications précédentes, le réceptacle (1) ayant une section en retrait (13) le long de l'une de ses parois latérales avec une profondeur suffisante pour accueillir le circuit de capteur capacitif (2) et le module électronique (5), de préférence à côté du circuit de capteur capacitif (2) ; et le dispositif (10) comprenant un couvercle (3) configuré pour fixer le circuit de capteur capacitif (2) dans ladite section en retrait (13) ; de préférence le dispositif (10) comprenant en outre un ou plusieurs éléments d'étanchéité (31) disposés entre le réceptacle (1) et le couvercle (3) de sorte que le fluide ne puisse pas atteindre le circuit de capteur capacitif (2) .

8. Dispositif (10) selon l'une des revendications précédentes, le volume intérieur du réceptacle étant effilé vers la surface inférieure (11).

9. Dispositif (10) selon l'une des revendications précédentes, les connecteurs électriques (26) étant sensiblement de la même longueur.

10. Dispositif (10) selon l'une des revendications précédentes, le plan de masse (23) étant hachuré.

11. Dispositif (10) selon l'une des revendications précédentes, l'unité de traitement étant configurée pour déterminer le volume d'urine en consultant la valeur correspondante dans une courbe d'étalonnage et/ou une table de consultation qui décrit la relation entre la hauteur du fluide et le volume du récipient qui termine ouvert.

12. Dispositif (10) selon l'une des revendications précédentes, le circuit de capteur capacitif (2) comprenant une électrode de capteur capacitif de référence qui est configurée pour déterminer une valeur de base indépendante de la présence d'un fluide dans le récipient (1) ; l'unité de traitement étant configurée pour recevoir la valeur de base de ladite électrode capacitive de référence et déterminer la présence et/ou l'absence de fluide dans le récipient (1) en comparant ladite valeur de base avec une valeur de capacité mesurée par une ou plusieurs électrodes de capteur de surface capacitives (21) ; de préférence, l'électrode de capteur capacitif de référence étant disposée entre les électrodes de capteur de surface capacitives (21) et le blindage électrique (4).

13. Dispositif (10) selon l'une quelconque des revendications précédentes, comprenant en outre un capteur de stabilité du fluide configuré pour déterminer la stabilité du fluide à l'intérieur du récipient (1) et l'unité de traitement étant configurée pour corriger les données de hauteur de fluide en retardant et/ou en répétant la mesure de la hauteur du fluide par le circuit de capteur capacitif (2) lorsqu'une instabilité du fluide est détectée ; de préférence, l'unité de traitement étant en outre configurée pour fournir un retour d'information à l'utilisateur afin de stabiliser le récipient (1) pour la mesure lorsqu'une instabilité du fluide est détectée ; de préférence, avec un indice audio et/ou visuel.

14. Dispositif (10) selon l'une quelconque des revendications précédentes, le dispositif de suivi d'urine (10) comprenant un capteur d'inclinaison configuré pour mesurer l'inclinaison du récipient (1) et l'unité de traitement étant configurée pour corriger les données de hauteur de fluide en déterminant l'angle et/ou la direction d'inclinaison et en convertissant les données de hauteur de fluide en données de hauteur de fluide corrigées ; de préférence en consultant les données de hauteur de fluide corrigées dans une courbe d'étalonnage et/ou une table de consultation qui décrit la relation entre la hauteur de fluide et l'angle et/ou la direction d'inclinaison du récipient.

15. Dispositif (10) selon l'une quelconque des revendications précédentes, le dispositif de suivi d'urine (10) comprenant un module de communication qui est configuré pour se connecter à un dispositif informatique (10), tel qu'un smartphone ou un ordinateur personnel, et envoyer le volume d'urine déterminé, de préférence le volume d'urine horodaté, audit dispositif informatique (10) ; et le dispositif de suivi d'urine (10) comprenant un module d'identification du sujet qui est configuré pour identifier le sujet lors de la connexion du dispositif de suivi d'urine (10) au dispositif informatique (10).
